# EUROPEAN PATENT APPLICATION

(11) **EP 4 821 440 A1**
(43) Date of publication of application: **14.10.2026**
(21) Application number: 25168762.0
(22) Date of filing: 07.04.2025
(51) Int. Cl.: A61B 6/03, A61B 6/00

(54) **CONTROLLING AN IMAGING DATA ACQUISITION**

(71) Applicant: Koninklijke Philips N.V., 5656 AE Eindhoven (NL)
(72) Inventor: SENEGAS, Julien Thomas, Eindhoven (NL); KRUEGER, Sascha, Eindhoven (NL); FRERKING, Lena Christina, Eindhoven (NL); VAN EE, Raymond, Eindhoven (NL)
(74) Representative: Philips Intellectual Property & Standards

(57) **Abstract**

Disclosed herein is a method for controlling an imaging data acquisition by a medical imaging system (110) from a region of interest (170) of a subject (118), which is at least partially moved due to respiratory movements of the subject (118). The method comprises receiving an imaging protocol (420) with a set of imaging parameters (422) for an executing of the imaging data acquisition. A respiratory signal of the subject (118) is received and a length (440) of a compliant respiratory phase (320) determined, during which the respiratory movements comply with a predefined movement tolerance (424). The length (440) is compared with an imaging time (426) required for acquiring the imaging data (470). The medical imaging system (110) is controlled to acquire the imaging data (470) based on a result of the comparing.

## Description

### TECHNICAL FIELD

The invention relates to the field of medical imaging, in particular it relates to an imaging data acquisition from a region of interest of a subject, which is at least partially moved due to respiratory movements of the subject.

### BACKGROUND

When acquiring imaging data from a region of interest of a breathing subject, which is at least partially moved due to respiratory movements of the subject, such movements may constitute a challenge for a reconstruction of medical images using the acquired imaging data. During normal breathing, parts of the breathing subject will move as the lungs fill and empty with air. These movements are referred to as respiratory movements of the subject and may cause issues, in particular for medical imaging procedures in which an image acquisition requires some interval of continuous measurements, such as computed tomography (CT) imaging and/or magnetic resonance imaging (MRI). Too strong respiratory movements during image data acquisition may lead to movement artefacts in the resulting reconstructed images. Due to the artifacts the images may, e.g., be vague and appear moved. An approach to overcome this challenge is to instruct the subject to hold his or her breath during the imaging data acquisition. However, this approach strongly depends on the individual ability of the subject to follow respective instructions and hold his or her breath. Therefore, holding the breath may not be a feasible approach for all subjects and cause problems.

### SUMMARY

The invention provides for a method for controlling an imaging data acquisition by a medical imaging system from a region of interest of a subject using a computational system with the region of interest being at least partially moved due to respiratory movements of the subject in the independent claims. The invention further provides for a computer program as well as a medical system for controlling an imaging data acquisition by a medical imaging system from a region of interest of a subject using a computational system with the region of interest being at least partially moved due to respiratory movements of the subject in the independent claims.

In one aspect, a method for controlling an imaging data acquisition by a medical imaging system from a region of interest of a subject using a computational system is disclosed. The region of interest is at least partially moved due to respiratory movements of the subject.

The method comprises receiving an imaging protocol defining a set of imaging parameters for an executing of the imaging data acquisition by the medical imaging system. The imaging protocol further is assigned with a predefined movement tolerance. The movement tolerance defines a maximum level of movement acceptable for the imaging data acquisition. The imaging data acquisition is assigned with an imaging time defining a time interval required for acquiring imaging data. The method comprises receiving a respiratory signal of the subject descriptive of the respiratory movements of the region of interest during respiratory cycles of the subject. The method comprises analyzing the respiratory signal. The analyzing comprises determining a motion index of respiratory movements of the region of interest and, using the motion index, a length of a compliant respiratory phase within the individual respiratory cycles, during which the respiratory movements of the region of interest comply with the predefined movement tolerance. The method comprises comparing the length of the compliant respiratory phase with the imaging time. The method comprises controlling the medical imaging system to acquire the imaging data from the region of interest based on a result of the comparing.

In another aspect, a computer program for controlling an imaging data acquisition by a medical imaging system from a region of interest of a subject with the region of interest being at least partially moved due to respiratory movements of the subject is disclosed. The computer program comprises machine executable instructions.

An execution of the machine executable instructions using a computational system causes the computational system to receive an imaging protocol defining a set of imaging parameters for an executing of the imaging data acquisition by the medical imaging system. The imaging protocol further is assigned with a predefined movement tolerance. The movement tolerance defines a maximum level of movement acceptable for the imaging data acquisition. The imaging data acquisition is assigned with an imaging time defining a time interval required for acquiring imaging data. The execution causes the computational system to receive a respiratory signal of the subject descriptive of the respiratory movements of the region of interest during respiratory cycles of the subject. The execution causes the computational system to analyze the respiratory signal. The analyzing comprises determining a motion index of respiratory movements of the region of interest and, using the motion index, a length of a compliant respiratory phase within the individual respiratory cycles, during which the respiratory movements of the region of interest comply with the predefined movement tolerance. The execution causes the computational system to compare the length of the compliant respiratory phase with the imaging time. The execution causes the computational system to control the medical imaging system to acquire the imaging data from the region of interest during based on a result of the comparing.

In another aspect, a medical system comprising a computational system for controlling an imaging data acquisition by a medical imaging system from a region of interest of a subject with the region of interest being at least partially moved due to respiratory movements of the subject is disclosed. The computational system comprises a memory storing machine executable instructions for an execution by a computational system.

The execution of the machine executable instructions causes the computational system to receive an imaging protocol defining a set of imaging parameters for an executing of the imaging data acquisition by the medical imaging system. The imaging protocol further is assigned with a predefined movement tolerance. The movement tolerance defines a maximum level of movement acceptable for the imaging data acquisition. The imaging data acquisition is assigned with an imaging time defining a time interval required for acquiring imaging data. The execution causes the computational system to receive a respiratory signal of the subject descriptive of the respiratory movements of the region of interest during respiratory cycles of the subject. The execution causes the computational system to analyze the respiratory signal. The analyzing comprises determining a motion index of respiratory movements of the region of interest and, using the motion index, a length of a compliant respiratory phase within the individual respiratory cycles, during which the respiratory movements of the region of interest comply with the predefined movement tolerance. The execution causes the computational system to compare the length of the compliant respiratory phase with the imaging time. The execution causes the computational system to control the medical imaging system to acquire the imaging data from the region of interest based on a result of the comparing.

### BRIEF DESCRIPTION OF THE DRAWINGS

In the following preferred embodiments of the invention will be described, by way of example only, and with reference to the drawings in which:
Fig. 1 shows a flow chart illustrating an exemplary method controlling an imaging data acquisition by a medical imaging system;
Fig. 2 shows a flow chart illustrating an exemplary method for analyzing a respiratory signal;
Fig. 3 shows an exemplary respiratory signal;
Fig. 4 shows an exemplary respiratory signal with a compliant respiratory phase indicated for some respiratory cycles;
Fig. 5 shows exemplary respiratory signals;
Fig. 6 shows exemplary respiratory signals;
Fig. 7 shows exemplary respiratory signals;
Fig. 8 shows exemplary respiratory signals;
Fig. 9 shows an exemplary computational system of an exemplary medical system;
Fig. 10 shows an exemplary medical system;
Fig. 11 shows an exemplary medical system comprising an exemplary medical imaging system in form of an MRI system; and
Fig. 12 shows an exemplary medical system comprising an exemplary medical imaging system in form of a CT system.

### DESCRIPTION OF EMBODIMENTS

Like numbered elements in these figures are either equivalent elements or perform the same function. Elements which have been discussed previously will not necessarily be discussed in later figures if the function is equivalent.

For example, a method for controlling an imaging data acquisition by a medical imaging system from a region of interest of a subject using a computational system is provided. The region of interest is at least partially moved due to respiratory movements of the subject.

The method comprises receiving an imaging protocol defining a set of imaging parameters for an executing of the imaging data acquisition by the medical imaging system. The imaging protocol further is assigned with a predefined movement tolerance. The movement tolerance defines a maximum level of movement acceptable for the imaging data acquisition. The imaging data acquisition is assigned with an imaging time defining a time interval required for acquiring imaging data. A respiratory signal of the subject descriptive of the respiratory movements of the region of interest during respiratory cycles of the subject is received. The respiratory signal is analyzed. The analyzing comprises determining a motion index of respiratory movements of the region of interest and, using the motion index, a length of a compliant respiratory phase within the individual respiratory cycles, during which the respiratory movements of the region of interest comply with the predefined movement tolerance. For example, the analyzing comprises analyzing a respiratory signal amplitude throughout the respiratory cycle in order to determine in which sub-phase or sub-phases of the respiratory cycle the respiratory movement, e.g., the respiratory movement per unit time, complies with the predefined movement tolerance, i.e., does not exceed the predefined movement tolerance. In case a signal sub-phase of the respiratory cycle is determined, the respective sub-phase is, e.g., identified as the compliant respiratory phase. In case a plurality of sub-phases of the respiratory cycle are determined, e.g., a longest one of the sub-phases is identified as the compliant respiratory phase. The length of the compliant respiratory phase is compared with the imaging time. The medical imaging system is controlled to acquire the imaging data from the region of interest based on a result of the comparing.

Examples may have the beneficial effect of allowing for a controlling of the acquiring of the imaging data based on a result of the comparing, i.e., depending on a relationship between the length of the compliant respiratory phase and the imaging time. In case that the imaging time is equal to or smaller than the length of the compliant respiratory phase, i.e., in case the imaging data acquisition using the imaging parameters defined by the imaging protocol fits into the compliant respiratory phase, the imaging data may, e.g., be acquired data during a single compliant respiratory phase using the imaging protocol with the imaging parameters. In case that the imaging time exceeds the length of the compliant respiratory phase, i.e., in case the imaging data acquisition using the imaging parameters defined by the imaging protocol does not fit into the compliant respiratory phase, the imaging parameters of the imaging protocol may, e.g., be adjusted and the imaging data may, e.g., be acquired data using the adjusted imaging protocol with the adjusted imaging parameters.

The motion index may e.g., be determined in form of a motion index signal, i.e., a time signal having a duration of at least one respiratory cycle and being descriptive of the respiratory movements of the region of interest over time. For example, the motion index signal may be determined by integrating incremental respiratory movements described by the respiratory signal over a time period during which a portion of the region of interest contributes to the acquired data. This time period may, e.g., be defined by the imaging parameters of the imaging protocol.

For example, a sliding window method may be used for determining the motion index signal.

For example, the sliding-window method may comprise calculating an integral of the incremental respiratory movements described by the respiratory signal for a subset of data points, i.e., values of the respiratory movements described by the respiratory signal, within a fixed-size window that slides over a dataset, i.e., the respiratory signal. For example, a sliding-window integral of the incremental respiratory movements, i.e., displacements, measured over a window of size w, may be determined for every point in the respiratory cycle. The result is again a time signal. A section of this time signal, within which a predefined threshold for the integrals described by the predefined movement tolerance of the imaging protocol is not exceeded, may be identified as the compliant respiratory phase. During this compliant respiratory phase, the respiratory movements comply with the predefined movement tolerance.

For example, the sliding-window method may comprise calculating an integral of an absolute value of the incremental respiratory movements described by the respiratory signal for a subset of data points, i.e., values of the respiratory movements described by the respiratory signal, within a fixed-size window that slides over a dataset, i.e., the respiratory signal. For example, a sliding-window integral of the absolute values of the incremental respiratory movements, i.e., displacements, measured over a window of size w, may be determined for every point in the respiratory cycle. The result is again a time signal. A section of this time signal, within which a predefined threshold for the integrals described by the predefined movement tolerance of the imaging protocol is not exceeded, may be identified as the compliant respiratory phase. During this compliant respiratory phase, the respiratory movements comply with the predefined movement tolerance.

For example, the sliding-window method may comprise calculating a standard variation of incremental respiratory movements described by the respiratory signal for a subset of data points, i.e., values of the respiratory movements described by the respiratory signal, within a fixed-size window that slides over a dataset, i.e., the respiratory signal. For example, a sliding-window standard deviation of the incremental respiratory movements, i.e., displacements, measured over a window of size w, may be determined for every point in the respiratory cycle. The result is again a time signal. A section of this time signal, within which a predefined threshold for the standard deviations described by the predefined movement tolerance of the imaging protocol is not exceeded, may be identified as the compliant respiratory phase. During this compliant respiratory phase, the respiratory movements comply with the predefined movement tolerance.

The size of the window used for the sliding window method may, e.g., be defined by the imaging parameters of the imaging protocol. The imaging parameters may, e.g., define a time period during which a portion of the region of interest contributes to the acquired data. This time period may, e.g., be used as the size of the window. Considering, e.g., a computed tomography imaging data acquisition using a CT system, a portion of the subject and thus a portion of the region of interest may remain within an X-ray beam of the CT system for a duration given by w = colSize*rotS/pitch defined by the following imaging parameters: a gantry rotation speed "rotS", a table pitch "pitch", and a collimation size "colSize". For example, the duration w may be used as the size of the window.

The motion index may e.g., be determined in form of a motion index measure, i.e., in form of a scalar determined for a sub-phase of the respiratory cycle. The scalar may, e.g., be provided by an integral of the incremental respiratory movements described by the respiratory signal during the respective sub-phase. The scalar may, e.g., be provided by an integral of absolute values of the incremental respiratory movements described by the respiratory signal during the respective sub-phase. The scalar may, e.g., be provided by a standard deviation of the incremental respiratory movements described by the respiratory signal during the respective sub-phase.

For example, in response to determining that the imaging time is equal to or smaller than the length of the compliant respiratory phase, the controlling of the medical imaging system to acquire the imaging data from the region of interest is a controlling to acquire the imaging data during a single compliant respiratory phase using the imaging protocol with the imaging parameters.

Examples may have the beneficial effect of allowing for an execution of the imaging data acquisition during the single compliant respiratory phase using the imaging protocol with the imaging parameters as received if the imaging time and thus the imaging data acquisition fits into the compliant respiratory phase.

For example, in response to determining that the imaging time exceeds the length of the compliant respiratory phase, the method further comprises determining adjustments of the imaging parameters for adjusting the imaging data acquisition to the length of the compliant respiratory phase. The imaging protocol is adjusted. The adjusting comprises adjusting imaging parameters of the imaging protocol using the determined adjustments. The controlling of the medical imaging system to acquire the imaging data from the region of interest is a controlling to acquire the imaging data from the region of interest during one or more compliant respiratory phases of one or more of the respiratory cycles using the adjusted imaging protocol with the adjusted imaging parameters.

Examples may have the beneficial effect of allowing for an adjusting of an imaging data acquisition to a respiratory behavior of a subject. In particular, a shallow breathing behavior may be detected in form of the compliant respiratory phase and the imaging data acquisition adjusted accordingly.

Examples may allow for a free-breathing imaging data acquisition, i.e., for an imaging data acquisition without a requirement for the subject to hold his or her breath. Examples may allow for avoiding problems resulting from a breath holding. The controlling of the medical imaging system to acquire the imaging data may, e.g., comprise starting the imaging data acquisition without a breath-hold commands. The controlling of the medical imaging system to acquire the imaging data may, e.g., comprise triggering a starting of the imaging data acquisition such that the imaging data acquisition fits in one or more compliant respiratory phases of the one or more of the respiratory cycles. Depending on the result of the comparing of the length of the compliant respiratory phase with the imaging time, the imaging data acquisition may, e.g., be executed using the imaging protocol without adjustments or using the adjusted imaging protocol with the adjusted imaging parameters. The controlling of the medical imaging system to acquire the imaging data may, e.g., comprise triggering a starting of the imaging data acquisition using the adjusted imaging protocol with the adjusted imaging parameters such that the imaging data acquisition fits in a single compliant respiratory phase of a single respiratory cycle.

Breath holding may, e.g., be highly inconvenient for the subject, in particular for a subject with breathing difficulties or a very young subject. A subject may, e.g., not be able to sustain the breath holding for a duration sufficiently long to acquire the imaging data. Restarting breathing, while imaging data is still acquired, may cause severe motion artifacts in images reconstructed using the acquired imaging data.

Moreover, when imaging data has to be acquired over a plurality of successive breathingholding phases, slices may, e.g., be mis-registered because the different breath-hold phases may differ from each other.

Examples may allow to determine adjustments for a given imaging protocol with a certain respiratory movement tolerance by monitoring a respiratory pattern of a respiratory signal of a subject. A compliant respiratory phase may be derived, i.e., an interval of a respiratory cycle, e.g., a regular cycle of the subject, in which respiratory movements caused by the breathing of the subject are within the respiratory movement tolerance. Furthermore, the medical imaging system may be controlled to acquire the imaging data using imaging parameters adjusted to the compliant respiratory phase. Thus, a need for a breath holding may be avoided.

The predefined movement tolerance assigned to the imaging protocol is, e.g., a predefined tolerance on movement per time unit. The predefined movement tolerance, i.e., the predefined tolerance on movements per time unit, e.g., defines a maximum level of movement acceptable per time unit for the imaging data acquisition. The predefined movement tolerance may, e.g., be a predefined movement tolerance determined based on the set of imaging parameters of the imaging protocol. For example, in case of a CT imaging data acquisition, the predefined movement tolerance may, e.g., be determined based on a rotation speed, a pitch, and/or a collimation length determined as imaging parameters of the imaging protocol.

For example, different types of adjustments may be determined and applied to the imaging parameters. Adjustments may result in a reduction of the imaging time, such that the imaging time and consequently the data acquisition fits into a single compliant respiratory phase. In case the imaging time exceeds the length of the compliant respiratory phase by a time span larger than the predefined time span, i.e., when adjustments required for fitting the data acquisition into a single compliant respiratory phase would be too strong, adjustments may be used to split the data acquisition to be executed during two or more compliant respiratory phases. The splitting may, e.g., be combined with additional adjustments for additionally reducing the imaging time. The reduction of the imaging time may result in a reduction of the number of compliant respiratory phases required for acquiring the imaging data in the split form. For example, the additional adjustments to reduce the imaging time may only be applied when the application results in a reduction of the number of compliant respiratory phases required for acquiring the imaging data. **In** case the imaging time exceeds the length of the compliant respiratory phase by a time span not only larger than the predefined time span, but also larger than a predefined maximum time span, the subject may be instructed to hold his or her breath and the data acquisition may be executed during the breath holding.

Examples may allow for determining, whether a total imaging data acquisition can be performed during a single compliant respiratory phase of a single respiratory cycle. This is the case, if the imaging time does not exceed the length of the compliant respiratory phase. In this case, e.g., the unadjusted imaging protocol may be used for the imaging data acquisition. For a given subject and a given imaging protocol, the breathing by the subject may, e.g., be light enough that a full respiratory cycle of the subject may comply with the predefined movement tolerance of the imaging protocol. In this case, the full respiratory cycle may qualify as a compliant respiratory phase and no adjustments of the imaging parameters of the imaging protocol may be required.

In case the compliant respiratory phase is slightly too short, i.e., if the imaging time exceeds the length of the compliant respiratory phase by a time span equal to or smaller than a predefined time span, the imaging parameters of the imaging protocol may, e.g., be adjusted automatically to make it fit.

If the imaging time exceeds the length of the compliant respiratory phase by a time span larger than the predefined time span, but still equal to or smaller than a predefined maximum time span, the imaging parameters of the imaging protocol may, e.g., be adjusted automatically to split the imaging data acquisition over more than one compliant respiratory phase.

If the imaging time exceeds the length of the compliant respiratory phase by a time span larger than the predefined maximum time span, the imaging data acquisition may, e.g., be executed using a breath holding approach. For this purpose, e.g., the unadjusted imaging protocol may be used and the subject may be instructed to hold his or her breath. The subject may be instructed to hold his or her breath for a few seconds, e.g., 8 to 15 seconds, and the imaging data acquisition may be executed during this breath-hold phase. If necessary to acquire all imaging data to be acquired, the data acquisition may even be split over several, successive breath-hold phases. In this case, the subject may, e.g., be instructed repeatedly to hold his or her breath.

In case a breath-hold scan is preferred because there is no sufficiently long compliant respiratory phase in the regular respiratory pattern of the subject, e.g., a timing of the auto-voice commands for the breath holding may be adjusted to match a waveform of the respiratory signal in real-time. For example, for a subject having a breathing interval of 4 seconds, an audio command instructing the subject to "breathe-in" may, e.g., be output 3 seconds after a latest peak detection within the respiratory signal, such that the subject would naturally start breathing in when hearing this command. Furthermore, e.g., a command "hold breath" may be output at the end of the breathing-in of the subject.

The respiratory signal may, e.g., be received from a respiratory sensor system comprising one or more respiratory sensors. For example, the respiratory sensor system comprising the one or more respiratory sensors may be used to automatically acquire the respiratory signal of the subject. The computational system may, e.g., be configured to determine at least semi-quantitatively a respiratory waveform of the respiratory signal of a subject. For example, a respiratory amplitude, i.e., an amplitude of respiratory movements, may be determined in representative units, such as mm, as well as a respiratory frequency. The respiratory signal may be used for detecting and assessing respiratory cycles of the subject, especially the compliant respiratory phase within the individual respiratory cycles, during which respiratory movements comply with the predefined movement tolerance. The compliant respiratory phase determined may, e.g., be an average compliant respiratory phase determined using a plurality of compliant respiratory phases detected for a plurality of respiratory cycles. The compliant respiratory phase determined may, e.g., be a shortest compliant respiratory phase of a plurality of compliant respiratory phases detected for a plurality of respiratory cycles.

When a compliant respiratory phase of sufficient length is determined, the respiratory signal may, e.g., be considered shallow breathing. A shallow breathing may allow for an imaging data acquisition without requiring a breath holding by the subject. For example, a respiratory cycle frequency or equivalently a respiratory cycle interval duration as well as an amplitude of the respiratory signal may be measured, e.g., in real-time. For this purpose, e.g., a peak detection algorithm and/or a trained classifier may be used. The aforementioned parameters may, e.g., be analyzed and an imaging data acquisition strategy adapted automatically to the result of this analysis. The adjusting of the imaging data acquisition may, e.g., comprise an adjusting of the imaging protocol. Especially, if the breathing can be classified as shallow breathing, meaning that an amount of respiratory motion present during a sufficiently long compliant respiratory phase is with a predefined movement tolerance assigned to the imaging protocol, a special form of free breathing imaging data acquisition may be performed: as many imaging data as possible may be acquired during the compliant respiratory phase, with the start of the imaging data acquisition being automatically triggered based on the real-time analysis of the respiratory cycle of the respiratory signal. In particular, the imaging data acquisition may be executed during a single respiratory cycle. Possibly, the imaging data acquisition may be split over a few respiratory cycles until all the imaging data to be acquired have been acquired, though it may be preferred to acquire all the imaging data within a cycle.

Examples may have the beneficial effect that no breath holding may be required during the imaging data acquisition. Examples may thus, e.g., be beneficial for pediatric applications by allowing to avoid a breath-hold.

The respiratory signal may be used for triggering the medical imaging data acquisition. The triggering may, e.g., be configured to acquire the medical imaging data at a certain point of time of a respiratory cycle of the subject and/or to acquire the medical imaging data at a state of minimum or even no breathing related movement of the subject. The latter may, e.g., reduce or even prevent motion artifacts. Furthermore, respiratory signal may, e.g., be used to determine breathing related movements of body parts of the subject. This may, e.g., support a focusing of imaging data acquisition on certain body parts by enabling a precise breathing dependent position determining and/or predicting of the same.

The respiratory signal may, e.g., be used for monitoring a vital state of the subject.

Medical imaging refers to a method of acquiring medical imaging data, e.g., interior imaging data of a subject, which may, e.g., be used for clinical analysis. Medical imaging methods may, e.g., comprise such methods as radiography, magnetic resonance imaging, ultrasound, computed tomography, etc.

Examples may allow to reduce and/or avoid motion artifacts due to respiration movements, when using a medical imaging method, like an MRI or CT scan, to acquire imaging data from a region of interest located in a torso of a subject, such as lung, heart, and/or liver. For such medical imaging methods an imaging time may, e.g., be a few seconds or even a few minutes long.

Examples may, e.g., comprise an analyzing of the respiratory signal of the subject over one or more respiratory cycles and determining, whether an imaging data acquisition using the imaging protocol can be performed during a regular respiratory cycle, i.e., during a single compliant respiratory phase of a regular respiratory cycle. For example, the imaging time during which a given portion of a body of the subject is providing signal to the acquired imaging may be compared with the length of the compliant respiratory phase. Based on this assessment, e.g., a starting point in the respiratory cycle for the data acquisition as well as a data acquisition duration may be determined.

For example, in response to determining that the imaging time exceeds the length of the compliant respiratory phase by a time span equal to or smaller than a predefined time span, the adjustments being determined comprise first adjustments of the imaging parameters for fitting the imaging data acquisition into a single compliant respiratory phase of a single respiratory cycle. The controlling of the medical imaging system to acquire the imaging data from the region of interest is a controlling to acquire the imaging data during a single compliant respiratory phase.

Examples may have the beneficial effect of enabling an imaging data acquisition within a single compliant respiratory phase of a single respiratory cycle. Acquiring imaging data during a single compliant respiratory phase may, e.g., have the beneficial effect that a long overall time to execute the overall imaging data acquisition may be avoided by executing the complete imaging acquisition within a single respiratory cycle. Furthermore, by avoiding a splitting of the imaging data acquisition over a plurality of respiratory cycles, motion artifacts or slice mis-registrations may be prevented from occurring in reconstructed images. Such motion artifacts and slice mis-registrations may result, if the respiration pattern of the subject is non-steady, e.g., comprising alternating short and long respiratory cycles and/or alternating respiratory cycles with shallow and deep breathing phases.

For example, the imaging protocol further is assigned with a set of potential adjustments of the imaging parameters of the imaging protocol for reducing the imaging time. The determining of the first adjustments of the imaging parameters comprises selecting one or more of the potential adjustments from the set of potential adjustments.

Examples may have the beneficial effect that the first adjustments may be determined automatically by selecting one or more of the potential adjustments already provided.

For example, the selecting comprises using a selection criterion. The selecting criterion may require a reduction of the imaging time resulting from using the selected potential adjustments to be a minimum reduction sufficient to fit the reduced imaging time into the single compliant respiratory phase.

Examples may have the beneficial effect that an unnecessary reduction of the imaging time may be avoided. A reduction of the imaging time may typically require at least some type of reduction of quality of the imaging data being acquired. Thus, by avoiding an unnecessary reduction of the imaging time an unnecessary reduction of quality of the imaging data may be avoided as well.

For example, the medical imaging system comprises a computed tomography system and the imaging data acquisition may be a computed tomography data acquisition.

For example, the imaging parameters being adjusted for reducing the imaging time comprising one or more of the following: a gantry rotation speed; a table pitch; a collimation size; a scan length.

For example, the imaging protocol may define a gantry rotation speed for the imaging data acquisition as an imaging parameter of the set of imaging parameters. For example, the gantry rotation speed defined by the imaging protocol may be increased.

The gantry rotation speed refers to how quickly a gantry of a computed tomography system, which, e.g., houses an X-ray tube and one or more detectors, completes one full 360° rotation around the subject. The gantry rotation speed is typically measured in seconds per rotation, e.g., 0.5 second or 1 second per rotation. A faster rotation speed may, e.g., lead to a shorter imaging time, reducing motion artifacts and improving comfort of the subject. A faster rotation may also improve temporal resolution, which is beneficial for imaging moving structures like the heart.

For example, the imaging protocol may define a table pitch for the imaging data acquisition as an imaging parameter of the set of imaging parameters. For example, the table pitch defined by the imaging protocol may be increased.

The table pitch is a parameter that defines how far the patient table moves per rotation of the gantry relative to the collimated beam width. It may, e.g., be calculated as table movement per rotation divided by total collimated beam width. In case of a pitch of one, there are no overlapping slices of acquired imaging data or gaps between slices. For a pitch larger than one, the scanning may be faster, but an image resolution may be lower.

For example, the imaging protocol may define a collimation size for the imaging data acquisition as an imaging parameter of the set of imaging parameters. For example, the collimation size defined by the imaging protocol may be increased.

Collimation size refers to the thickness of an X-ray beam used to acquire imaging data, which directly affects slice thickness. The collimation size may be equal to a number of slices times a slice thickness. The collimation size influences the coverage per rotation. For a wider collimation, more of the subject's anatomy may be covered in a single rotation, potentially reducing the total number of rotations required for a given scan length.

For example, the imaging protocol may define a scan length for the imaging data acquisition as an imaging parameter of the set of imaging parameters. For example, the scan length defined by the imaging protocol may be decreased. The scan length is a total distance along the subject's body that is covered during the CT imaging data acquisition.

Using a CT system, the imaging data acquisition may, e.g., be performed in form of a helical scan. Such a helical scan may, e.g., be defined by the following imaging parameters: a gantry rotation speed "rotS", a table pitch "pitch", and a collimation size "colSize". A portion of a subject may remain within an X-ray beam of the CT system for a duration given by w = colSize*rotS/pitch. For example, a sliding-window average of an absolute respiratory displacement measured over a window of size "w" may be determined for every point of a respiratory cycle. The result is again a time signal. A section of this time signal, within which a predefined threshold described by a predefined movement tolerance of an imaging protocol, for example 5 mm, is not exceeded, may be identified as a compliant respiratory phase. During this compliant respiratory phase, the respiratory movements comply with the predefined movement tolerance. If the compliant respiratory phase is of a sufficient length, the breathing described by the respiratory signal may, e.g., be considered as shallow for the particular imaging protocol. Examples may also allow determining a start and/or end point of the imaging data acquisition during the respiratory cycle using a start and/or end point of the compliant respiratory phase within the respiratory cycle. For example, the imaging parameters table pitch and rotation speed may be adjusted for adjusting the imaging protocol such that a resulting adjusted imaging time fits within the determined compliant respiratory phase.

For example, the medical imaging system comprises a magnetic resonance imaging system. The imaging data acquisition may be a magnetic resonance imaging data acquisition.

For example, the imaging parameters being adjusted for reducing the imaging time comprise one or more of the following: an acquisition matrix size; a sampling rate; a field of view.

For example, the imaging protocol may define an acquisition matrix size for the imaging data acquisition as an imaging parameter of the set of imaging parameters. For example, the acquisition matrix size defined by the imaging protocol may be increased. The acquisition matrix size defines the number of data points acquired in each imaging direction, e.g., frequency and phase encoding directions. When combined with a field of view, it may determine an in-plane voxel size, i.e., resolution. For example, if the field of view is fixed, a smaller matrix size may lead to larger voxels and a lower resolution.

For example, the imaging protocol may define a sampling rate for the imaging data acquisition as an imaging parameter of the set of imaging parameters. For example, the sampling rate defined by the imaging protocol may be decreased. The sampling rate determines how densely k-space lines are sampled during a readout of the respective lines. Adjusting the sampling rate may affect the resolution.

For example, the imaging protocol may define a field of view for the imaging data acquisition as an imaging parameter of the set of imaging parameters. For example, the field of view defined by the imaging protocol may be decreased. The field of view in a third dimension represents a total physical length over which imaging data is acquired. This may, e.g., be determined or be equal to the region of interest. A voxel size may, e.g. be given by the field of view divided by the matrix size in that dimension.

For example, the respiratory signal is a spatially resolved signal descriptive of a spatial distribution of the respiratory movements within the region of interest over time. The imaging parameters define a spatial sequence of the imaging data acquisition within the region of interest. The determining of the first adjustments of the imaging parameters comprises an adjusting of the spatial sequence of the imaging data acquisition defined by the imaging parameters depending on the spatial distribution of the respiratory movements.

Examples may, e.g., allow for adjusting an ordering of slices of imaging data to be acquired. For example, imaging data of motion-sensitive slices, i.e., slices of the region of interest within which comparably large respiratory movements occur during the compliant respiratory phase, may be acquired during at a point of time within the compliant respiratory phase, when the respiratory movements within these slices are the smallest. For example, for a CT helical scan, a couch may move in two different directions, i.e., in and out. For a lung scan, e.g., it may therefore be beneficial to acquire imaging data from the lower part of the lung, where the lung apices and the diaphragm are located, at a point in time during the compliant respiratory phase with little and/or with as little respiratory movements within the region of interest as possible, and to scan the upper part of the lung, which is close to the shoulders and therefore positionally more stable, at a point in time during the compliant respiratory phase with more respiratory movements within the region of interest. The upper part may, e.g., be scanned just after a peak of the respiratory cycle corresponding to a maximum exhaling, while the lower part may, e.g., be scanned towards the end of the exhale phase.

For example, the adjusting of the spatial sequence of the imaging data acquisition comprises adjusting a start position of the imaging data acquisition within the region of interest. A start position may, e.g., be adjusted such that the data acquisition starts at an end of the region of interest, which has the smallest respiratory movements at the beginning of the compliant respiratory phase.

For example, the adjusting of the spatial sequence of the imaging data acquisition comprises adjusting a direction along which the imaging data acquisition is successively executed. The imaging data acquisition may be executed along a predefined direction. Adjusting the direction may comprise deciding, whether the imaging data acquisition is successively executed in the respective direction or against the respective direction. The adjusting of the direction may, e.g., depend on a selection of start position of the imaging data acquisition within the region of interest.

For example, the adjusting of the spatial sequence of the imaging data acquisition comprises jumping between different positions within the region of interest for the imaging data acquisition. This may, in particular be the case for an MRI data acquisition. The positions may be selected such that for each position or at least for a majority of the positions a point in time during the compliant respiratory phase for the imaging data acquisition is elected, at which respiratory movements of at the respective position are minimal during the compliant respiratory phase.

For example, in response to determining that the imaging time exceeds the length of the compliant respiratory phase by a time span larger than a predefined time span, the adjustments being determined comprise second adjustments of the imaging parameters for splitting the imaging data acquisition into two or more imaging data acquisition sets to be acquired during two or more compliant respiratory phases of two or more of the respiratory cycles. Each of the acquisition sets is configured to require an execution time fitting into one of the respective compliant respiratory phases. The controlling of the medical imaging system to acquire the imaging data from the region of interest is a controlling to acquire the imaging data during the two or more compliant respiratory phases of the two or more respiratory cycles.

Examples may have the beneficial effect that the imaging data acquisition may be split over a plurality of compliant respiratory phases. Thus, the imaging data acquisition may be executed within compliant respiratory phases without requiring the imaging time to be reduced or without requiring the imaging time to be reduced too much.

Examples may allow the subject to breathe freely, while a respiratory signal of the subject is acquired and monitored by means of a dedicated respiration sensor system. The imaging data acquisition and/or image reconstruction may then be synchronized with the respiratory cycles of the subject using the respiratory signal, such as to acquire the imaging data and/or use imaging data that correspond to the compliant respiratory phases of a plurality of, e.g., successive respiratory cycles.

For example, in addition to the second adjustments also first adjustments may be determined. Thus, the imaging data acquisition may not only be split, but in addition the imaging time may be reduced. In this case the first adjustments may, e.g., be determined to fit the imaging data acquisition into a smaller and/or smallest possible plurality of compliant respiratory phases.

For example, in response to the determining that the imaging time exceeds the length of the compliant respiratory phase by a time span larger than the predefined time span, the adjustments being determined additionally comprise third adjustments of the imaging parameters of the imaging protocol for fitting a maximum portion of the imaging data acquisition into a first acquisition set of the two or more acquisition sets acquired during a first compliant respiratory phase of a first respiratory cycle of the two or more respiratory cycles.

Examples may have the beneficial effect that as much imaging data as possible may be acquired within a single compliant respiratory phase. Thus, influences of differences between compliant respiratory phases of different respiratory cycles may be reduced and/or minimized. For example, the third adjustments may correspond to the aforementioned first adjustments to reduce the imaging time. The third adjustments are focused on increasing the amount of imaging data acquired during the first compliant respiratory phase. This does not necessarily result in a reduction of the number of compliant respiratory phases during which the imaging data acquisition is executed, but it may.

For example, the predefined time span is a predefined fraction of the imaging time.

For example, the predefined fraction is 5% to 20% of the imaging time. For example, the predefined fraction is 7% to 15% of the imaging time. For example, the predefined fraction is 10% of the imaging time.

For example, the predefined time span is a compensable time span assigned to the imaging protocol, which defines a maximum time span by which the imaging time is reducible using the set of potential adjustments of the imaging parameters of the imaging protocol.

For example, the predefined maximum time span is equal to three times, four times or five times the imaging time. For example, the predefined maximum time span is equal to three times, four times or five times the compensable time span.

For example, the analyzing of the respiratory signal further comprises determining a start time of the compliant respiratory phases within the respiratory cycles. Using the start time, a trigger comprised by the respiratory cycles within the respiratory signal is determined for triggering the imaging data acquisition in time with the start of the compliant respiratory phase. The controlling of the medical imaging system comprises monitoring the respiratory signal and triggering the acquiring of the imaging data in response to detecting the trigger in the respiratory signal. The acquiring of the imaging data may, e.g., be triggered such that the imaging data acquisition fits in a single compliant respiratory phase of a single respiratory cycle.

Examples may have the beneficial effect that an automated execution of the imaging data acquisition may be implemented.

For example, the analyzing of the respiratory signal further comprising determining an amplitude of the signal. For example, the analyzing of the respiratory signal further comprising determining a respiratory cycle interval duration time. For example, the analyzing of the respiratory signal further comprising determining a respiratory cycle frequency. For the analyzing, e.g., a peak detection algorithm and/or a trained classifier being used.

For example, the analyzing comprises analyzing the amplitude of the respiratory signal throughout the respiratory cycle in order to determine in which sub-phase or sub-phases of the respiratory cycle the respiratory movement, e.g., the respiratory movement per unit time, complies with the predefined movement tolerance, i.e., does not exceed the predefined movement tolerance. In case a signal sub-phase of the respiratory cycle is determined, the respective sub-phase is, e.g., identified as the compliant respiratory phase. In case a plurality of sub-phases of the respiratory cycle are determined, e.g., a longest one of the sub-phases is identified as the compliant respiratory phase. For example, the motion index may be used for analyzing the amplitude of the respiratory signal as described above.

For example, the trigger is a peak of the respective respiratory cycle within the respiratory signal. For example, a time difference between the trigger and the start time of the compliant respiratory phase is determined. For example, the triggering of the acquiring of the imaging data comprises a time-delayed starting of the imaging data acquisition relative to the trigger, such that the imaging data acquisition is started in time with the start of the compliant respiratory phase. For example, the starting of the imaging data acquisition is time-delayed by the time difference.

For example, the determining of the compliant respiratory phase comprises using a sliding-window average of the absolute respiratory movements. The sliding-window average refers to a calculating of an average of a subset of data points within a fixed-size window that slides over a dataset. For example, a sliding-window average of the absolute respiratory movements, i.e., displacements, measured over a window of size w, may be determined for every point in a respiratory cycle. The result is again a time signal. A section of this time signal, within which a predefined threshold described by a predefined movement tolerance of an imaging protocol, for example 5mm, is not exceeded, may be identified as a compliant respiratory phase. During this compliant respiratory phase, the respiratory movements comply with the predefined movement tolerance. Alternatively, e.g., a sliding-window integral, a sliding-window integral of absolute values, or a sliding-window standard deviation may be used for determining the compliant respiratory phase.

For example, a computer program for controlling an imaging data acquisition by a medical imaging system from a region of interest of a subject with the region of interest being at least partially moved due to respiratory movements of the subject is disclosed. The computer program comprises machine executable instructions. An execution of the machine executable instructions using a computational system causes the computational system to perform the method according to any of the examples described herein.

For example, the execution of the machine executable instructions causes the computational system to receive an imaging protocol defining a set of imaging parameters for an executing of the imaging data acquisition by the medical imaging system. The imaging protocol further is assigned with a predefined movement tolerance. The movement tolerance defines a maximum level of movement acceptable for the imaging data acquisition. The imaging data acquisition is assigned with an imaging time defining a time interval required for acquiring imaging data. A respiratory signal of the subject descriptive of the respiratory movements of the region of interest during respiratory cycles of the subject is received. The respiratory signal is analyzed. The analyzing comprises determining a length of a compliant respiratory phase within the individual respiratory cycles, during which the respiratory movements of the region of interest comply with the predefined movement tolerance. The length of the compliant respiratory phase is compared with the imaging time. In response to determining that the imaging time exceeds the length of the compliant respiratory phase, adjustments of the imaging parameters for adjusting the imaging data acquisition to the length of the compliant respiratory phase are determined. The imaging protocol is adjusted. The adjusting comprises adjusting imaging parameters of the imaging protocol using the determined adjustments. The medical imaging system is controlled to acquire the imaging data from the region of interest during one or more compliant respiratory phases of one or more of the respiratory cycles using the adjusted imaging protocol with the adjusted imaging parameters.

For example, a computer program product for controlling an imaging data acquisition by a medical imaging system from a region of interest of a subject with the region of interest being at least partially moved due to respiratory movements of the subject is disclosed. The computer program product may comprise a non-transitory computer readable storage medium having program instructions embodied therewith for execution by a computational system. An execution of the machine executable instructions causes the computational system to perform the method according to any of the examples described herein.

For example, the execution of the machine executable instructions causes the computational system to receive an imaging protocol defining a set of imaging parameters for an executing of the imaging data acquisition by the medical imaging system. The imaging protocol further is assigned with a predefined movement tolerance. The movement tolerance defines a maximum level of movement acceptable for the imaging data acquisition. The imaging data acquisition is assigned with an imaging time defining a time interval required for acquiring imaging data. A respiratory signal of the subject descriptive of the respiratory movements of the region of interest during respiratory cycles of the subject is received. The respiratory signal is analyzed. The analyzing comprises determining a length of a compliant respiratory phase within the individual respiratory cycles, during which the respiratory movements of the region of interest comply with the predefined movement tolerance. The length of the compliant respiratory phase is compared with the imaging time. In response to determining that the imaging time exceeds the length of the compliant respiratory phase, adjustments of the imaging parameters for adjusting the imaging data acquisition to the length of the compliant respiratory phase are determined. The imaging protocol is adjusted. The adjusting comprises adjusting imaging parameters of the imaging protocol using the determined adjustments. The medical imaging system is controlled to acquire the imaging data from the region of interest during one or more compliant respiratory phases of one or more of the respiratory cycles using the adjusted imaging protocol with the adjusted imaging parameters.

For example, a medical system comprising a computational system for controlling an imaging data acquisition by a medical imaging system from a region of interest of a subject with the region of interest being at least partially moved due to respiratory movements of the subject is provided. The computational system comprises a memory storing machine executable instructions for an execution by a computational system. The execution of the machine executable instructions causes the computational system to perform the method according to any of the examples described herein.

For example, the execution of the machine executable instructions causes the computational system to receive an imaging protocol defining a set of imaging parameters for an executing of the imaging data acquisition by the medical imaging system. The imaging protocol further is assigned with a predefined movement tolerance. The movement tolerance defines a maximum level of movement acceptable for the imaging data acquisition. The imaging data acquisition is assigned with an imaging time defining a time interval required for acquiring imaging data. A respiratory signal of the subject descriptive of the respiratory movements of the region of interest during respiratory cycles of the subject is received. The respiratory signal is analyzed. The analyzing comprises determining a length of a compliant respiratory phase within the individual respiratory cycles, during which the respiratory movements of the region of interest comply with the predefined movement tolerance. The length of the compliant respiratory phase is compared with the imaging time. In response to determining that the imaging time exceeds the length of the compliant respiratory phase, adjustments of the imaging parameters for adjusting the imaging data acquisition to the length of the compliant respiratory phase are determined. The imaging protocol is adjusted. The adjusting comprises adjusting imaging parameters of the imaging protocol using the determined adjustments. The medical imaging system is controlled to acquire the imaging data from the region of interest during one or more compliant respiratory phases of one or more of the respiratory cycles using the adjusted imaging protocol with the adjusted imaging parameters.

For example, the medical system further comprises the medical imaging system. For example, the medical imaging system is a magnetic resonance imaging system. For example, the medical imaging system is a computed tomography system.

For example, the medical system further comprises a respiratory sensor system configured for acquiring the respiratory signal of the subject.

For example, the respiratory sensor system comprises one or more contactless sensors. For example, the respiratory sensor system comprises a two-dimensional optical sensor system with one or more optical sensors. For example, one or more of the optical sensors are configured for detecting electromagnetic radiation in the visible spectrum. For example, one or more of the optical sensors are RGB-sensors configured for detecting red (R), green (G), and blue (B) light. For example, one or more of the optical sensors are configured for detecting electromagnetic radiation beyond the visible spectrum. For example, one or more of the optical sensors are IR-sensors configured for detecting infrared (IR) light. For example, one or more of the optical sensors are depth sensors configured for acquiring depth data. For example, the respiratory sensor system comprises one or more ultrasound sensors. For example, the respiratory sensor system comprises one or more radar sensors. For example, the respiratory sensor system comprises one or more radiofrequency (RF) sensors. For example, the medical imaging system is configured as a respiratory sensor system.

For example, the respiratory sensor system and a corresponding processing unit, e.g., a computational component of the computational system, are configured to measure at least semi-quantitatively a respiratory waveform of the respiratory signal of the subject. The respiratory signal acquired by the respiratory sensor system may, e.g., enable the computational system to determine a respiratory amplitude in representative units, such as mm, and a respiratory frequency.

For example, the medical imaging system is a magnetic resonance imaging (MRI) system configured for acquiring MRI navigator data used as the respiratory signal. For acquiring the MRI navigator data, e.g., a navigator pulse sequence is used.

For example, the medical imaging system is a computed tomography (CT) system configured for acquiring CT navigator data used as the respiratory signal.

For example, the respiratory sensor system comprises one or more contact-based sensors. For example, the one or more contact-based sensors are provided in form of pressure sensors. For example, the pressure sensors are provided in form of a two-dimensional sensor array. For example, the two-dimensional sensor array is integrated in a pressure sensing mattress. For example, the one or more contact-based sensors comprise a respiratory belt. For example, the one or more contact-based sensors comprise a respiratory bellows.

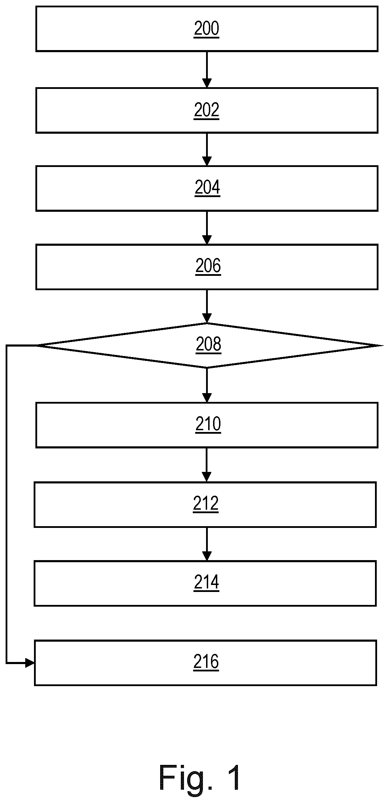

Fig. 1 shows an exemplary method for controlling an imaging data acquisition by a medical imaging system from a region of interest of a subject using a computational system. The region of interest is at least partially moved due to respiratory movements of the subject.

In block 200, an imaging protocol defining a set of imaging parameters for an executing of the imaging data acquisition by the medical imaging system is received. The imaging protocol further is assigned with a predefined movement tolerance. The movement tolerance defines a maximum level of movement acceptable for the imaging data acquisition. The imaging data acquisition is assigned with an imaging time defining a time interval required for acquiring imaging data.

In block 202, a respiratory signal of the subject descriptive of the respiratory movements of the region of interest during respiratory cycles of the subject is received. In block 204, the respiratory signal is analyzed. The analyzing comprises determining a motion index of respiratory movements of the region of interest and, using the motion index, a length of a compliant respiratory phase within the individual respiratory cycles, during which the respiratory movements of the region of interest comply with the predefined movement tolerance. For example, the determining of the compliant respiratory phase comprises using a sliding-window average of the absolute respiratory movements.

In block 206, the length of the compliant respiratory phase is compared with the imaging time. In block 208, it is checked, whether the imaging time exceeds the length of the compliant respiratory phase. If it is determined that the imaging time exceeds the length of the compliant respiratory phase, the method continues in block 210. Else, i.e., if it is determined that the imaging time does not exceed the length of the compliant respiratory phase, the method continues in block 216.

In block 210, in response to determining that the imaging time exceeds the length of the compliant respiratory phase, adjustments of the imaging parameters for adjusting the imaging data acquisition to the length of the compliant respiratory phase are determined.

For example, it may be checked, whether the imaging time exceeds the length of the compliant respiratory phase by a time span equal to or smaller than a predefined time span. For example, the predefined time span is a predefined fraction of the imaging time. For example, the predefined fraction is 5% to 20% of the imaging time, preferably 7% to 15% of the imaging time, and most preferably 9% to 11% of the imaging time.

In response to determining that the imaging time exceeds the length of the compliant respiratory phase by a time span equal to or smaller than the predefined time span, the adjustments being determined may comprise first adjustments of the imaging parameters for fitting the imaging data acquisition into a single compliant respiratory phase of a single respiratory cycle.

The imaging protocol may, e.g., be assigned with a set of potential adjustments of the imaging parameters of the imaging protocol for reducing the imaging time, from which one or more of the potential adjustments are selected as the adjustments to be used for adjusting the imaging parameters. For the selecting, e.g., a selection criterion may be used, which requires a reduction of the imaging time resulting from using the selected potential adjustments to be a minimum reduction sufficient to fit the reduced imaging time into the single compliant respiratory phase. Thus, an unnecessary reduction of the imaging time may be avoided. The reduction may rather be adjusted to match the compliant respiratory phase precisely.

For example, the predefined time span is a compensable time span assigned to the imaging protocol, which defines a maximum time span by which the imaging time is reducible using the set of potential adjustments of the imaging parameters of the imaging protocol.

In response to determining that the imaging time exceeds the length of the compliant respiratory phase by a time span larger than the predefined time span, the adjustments being determined may comprise second adjustments of the imaging parameters for splitting the imaging data acquisition into two or more imaging data acquisition sets to be acquired during two or more compliant respiratory phases of two or more of the respiratory cycles. Each of the acquisition sets is configured to require an execution time fitting into one of the respective compliant respiratory phases.

In response to determining that the imaging time exceeds the length of the compliant respiratory phase by a time span larger than the predefined time span, the adjustments being determined may additionally comprise third adjustments of the imaging parameters of the imaging protocol for fitting a maximum portion of the imaging data acquisition into a first acquisition set of the two or more acquisition sets acquired during a first compliant respiratory phase of a first respiratory cycle of the two or more respiratory cycles.

In block 212, the imaging protocol is adjusted. The adjusting comprises adjusting imaging parameters of the imaging protocol using the determined adjustments. In case of a computed tomography system, e.g., one or more of the following imaging parameters may be adjusted: a gantry rotation speed; a table pitch; a collimation size; a scan length. In case of a magnetic resonance imaging system, e.g., one or more of the following imaging parameters may be adjusted: an acquisition matrix size; a sampling rate; a field of view.

For example, the respiratory signal may be a spatially resolved signal, which is descriptive of a spatial distribution of the respiratory movements within the region of interest over time. Furthermore, the imaging parameters define a spatial sequence of the imaging data acquisition within the region of interest. The determining of the first adjustments of the imaging parameters comprises an adjusting of the spatial sequence of the imaging data acquisition defined by the imaging parameters depending on the spatial distribution of the respiratory movements. For example, the adjusting of a spatial sequence of the imaging data acquisition may comprise one or more of the following: adjusting a start position of the imaging data acquisition within the region of interest; adjusting a direction along which the imaging data acquisition is successively executed; jumping between different positions within the region of interest for the imaging data acquisition.

In block 214, the medical imaging system is controlled to acquire the imaging data from the region of interest during one or more compliant respiratory phases of one or more of the respiratory cycles using the adjusted imaging protocol with the adjusted imaging parameters. For controlling the acquiring of the imaging data, a trigger may be used. The analyzing of the respiratory signal in block 204 may further comprise determining a start time of the compliant respiratory phases within the respiratory cycles. Using the start time, the trigger comprised by the respiratory cycles within the respiratory signal may be determined and used for triggering the imaging data acquisition in time with the start of the compliant respiratory phase. For this purpose, the medical imaging system may be monitored and the acquiring of the imaging data triggered in response to detecting the trigger in the respiratory signal.

For example, the analyzing of the respiratory signal further comprising determining an amplitude of the signal. For example, the analyzing of the respiratory signal further comprising determining a respiratory cycle interval duration time. For example, the analyzing of the respiratory signal further comprising determining a respiratory cycle frequency. For the analyzing, e.g., a peak detection algorithm and/or a trained classifier being used. For example, the trigger is a peak of the respective respiratory cycle within the respiratory signal. For example, a time difference between the trigger and the start time of the compliant respiratory phase is determined. For example, the triggering of the acquiring of the imaging data comprises a time-delayed starting of the imaging data acquisition relative to the trigger, such that the imaging data acquisition is started in time with the start of the compliant respiratory phase. For example, the starting of the imaging data acquisition is time-delayed by the time difference.

If it has been determined that the imaging time exceeds the length of the compliant respiratory phase by a time span equal to or smaller than the predefined time span, the medical imaging system may, e.g., be controlled to acquire the imaging data during a single compliant respiratory phase.

If it has been determined that the imaging time exceeds the length of the compliant respiratory phase by a time span larger than the predefined time span, the medical imaging system may, e.g., be controlled to acquire the imaging data during the two or more compliant respiratory phases of the two or more respiratory cycles.

In block 216, in response to determining that the imaging time does not exceed the length of the compliant respiratory phase, the medical imaging system is controlled to acquire the imaging data from the region of interest during a single compliant respiratory phase using the imaging protocol without adjustments, i.e., the imaging protocol received in block 200.

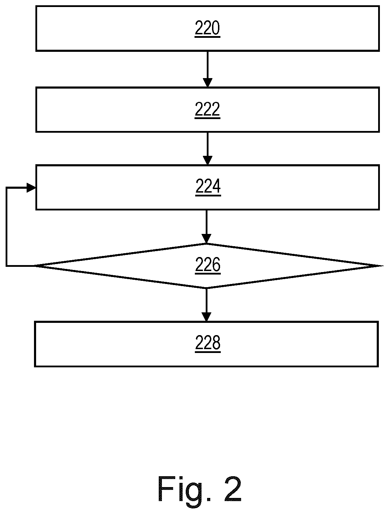

Fig. 2 shows an exemplary method for analyzing of a respiratory signal. In block 220, a start time of the compliant respiratory phases within the respiratory cycles is determined. In block 204, using the start time, a trigger comprised by the respiratory cycles within the respiratory signal is determined for triggering the imaging data acquisition in time with the start of the compliant respiratory phase. In block 224, the respiratory signal is monitored. In block 226, it is checked, whether the trigger is detected within the respiratory signal. If the trigger is detected within the respiratory signal, the method continues in block 228. Else, i.e., as long as the trigger is not detected within the respiratory signal, the monitoring of the respiratory signal in block 224 may be continued. In block 228, in response to detecting the trigger in the respiratory signal, the acquiring of the imaging data, i.e., the imaging data acquisition, is triggered. Depending on whether the imaging time assigned to the imaging protocol exceeds the length of the compliant respiratory phase or not, either an adjusted imaging protocol with adjusted imaging parameters or the original imaging protocol with the original imaging parameters may be used.

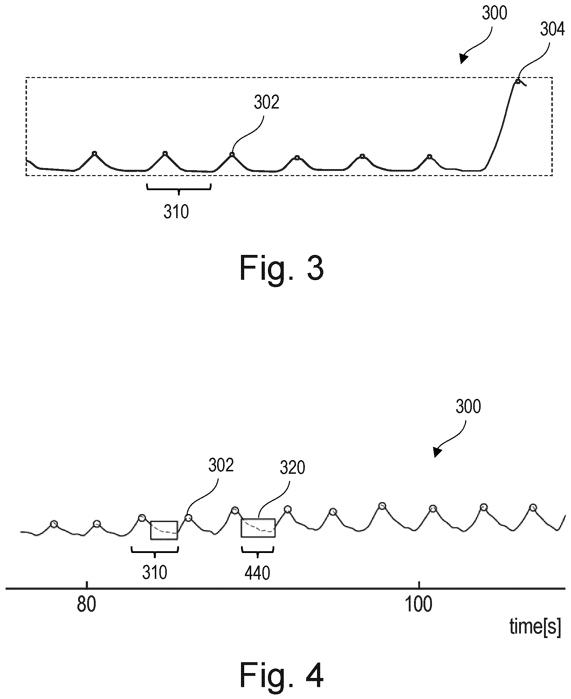

Fig. 3 shows an exemplary respiratory signal 300 detected using a respiratory sensor system comprising one or more optical sensors. Such a respiratory signal 300 may, e.g., be detected automatically using the respiratory sensor system monitoring respiratory movements of a subject. The first seven respiratory cycles 310 of the respiratory signal 300 shown in Fig. 3 comprise respiratory movements, which are significantly smaller than the eighth respiratory cycle 310. In particular the peaks 302 of the first seven respiratory cycles 310 are smaller than the peak 304 of the eighth respiratory cycle 310. Peaks 302, 304 of the respiratory cycles 310 are indicated in Fig. 3 by circles. These first seven respiratory cycles 310 may be classified as shallow breathing, i.e., they may comprise a compliant respiratory phase. Such a compliant respiratory phase is a phase of a respiratory cycle 310, during which the respiratory movements comply with the predefined movement tolerance. In other words, sizes of the respiratory movements during the compliant respiratory phase may not exceed a predefined threshold defined by the movement tolerance. Depending on the predefined movement tolerance, it may even be possible that the whole respiratory cycle 310 corresponds to a compliant respiratory phase, i.e., complies with the predefined movement tolerance. In the example shown, the first seven respiratory cycles 310 are followed by an eighth respiratory cycle of a deep inhalation. A respiratory rate detected for the respiratory signal is 18 Hz in the example of Fig. 3. This respiratory rate corresponds to a respiratory interval of 3.3 sec.

Fig. 4 shows another exemplary respiratory signal 300, which comprises a plurality of respiratory cycles 310. The respiratory cycles 310 each comprise a compliant respiratory phase 320, which are indicated by the boxes in Fig. 4. During such a compliant respiratory phase 320, the respiratory movements comply with the predefined movement tolerance. In other words, sizes of the respiratory movements during the compliant respiratory phase 320 may not exceed a predefined threshold defined by the movement tolerance. The respiratory signal 300 of Fig. 4 may, e.g., be classified as a shallow breathing signal. A respiratory signal 300 is, e.g., classified as a shallow breathing signal, when it comprises a compliant respiratory phase 320 with a length 440 corresponding to a predefined fraction of an imaging time required for acquiring imaging data. The respiratory signal 300 is, e.g., classified as a shallow breathing signal, when the length 440 of compliant respiratory phase 320 corresponds to at least 80%, at least 85%, or at least 90% of the imaging time. The circles depict the peaks 302 of the respiratory cycles 310, which correspond to the points of maximal inhalation. The compliant respiratory phase 320 shown as overlay boxes for two exemplary respiratory cycles 310 are predicted data acquisition intervals, which are suited for a data acquisition complying with the predefined movement tolerance.

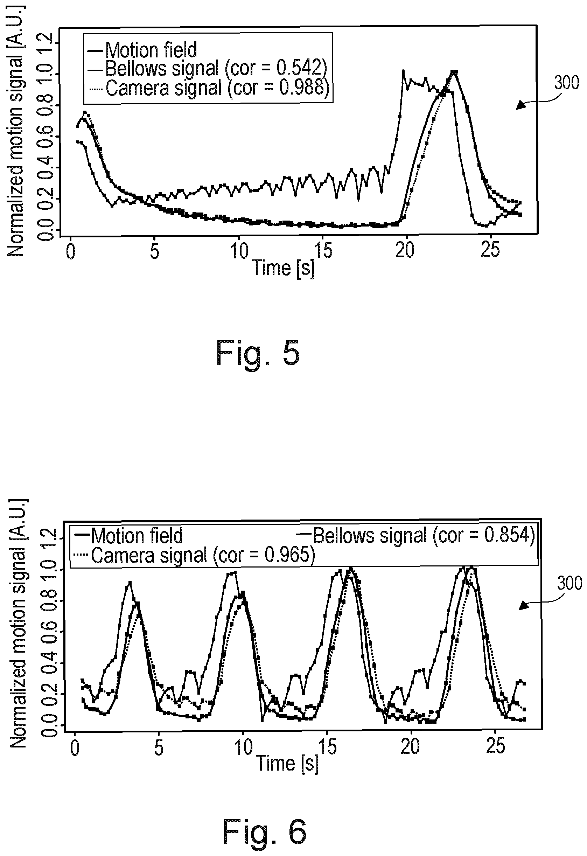

Fig. 5 shows exemplary respiratory signals 300 exhibiting shallow breathing, i.e., respiratory cycles with a compliant respiratory phase of a sufficient length. The sufficient length may, e.g., be defined as a length of at least 80%, at least 85%, or at least 90% of the imaging time. An automatically controlled image data acquisition may, e.g., use the respiratory signals 300 for triggering a start of the imaging data acquisition such that the start of the imaging data acquisition is synchronized with a start of the compliant respiratory phase. For example, a peak of a respiratory cycle of the respiratory signals 300 may be used as a trigger. The respiratory signals 300 may be monitored and the occurrence of a peak detected. A time difference between an occurrence of a peak of a respiratory cycle and a start of the compliant respiratory phase of the respective respiratory cycle 300 may, e.g., be two seconds. Thus, the imaging data acquisition may, e.g., be started automatically time-delayed by the time difference, e.g., two seconds, after peak detection. A length of the compliant respiratory phase of the respiratory cycles in Fig. 5 may, e.g., be 15 seconds. In case, the imaging data acquisition requires an imaging time to be executed, which is equal to or shorter than the length of the compliant respiratory phase, e.g., 15 seconds, the imaging data acquisition may be executed without adjustments of the imaging parameters. In case, the imaging time exceeds the length of the compliant respiratory phase, the imaging parameters for the imaging data acquisition may be adjusted to the length of the compliant respiratory phase. The imaging data acquisition may in particular be executed without requiring a breath-holding by the subject. In Fig. 5, a motion field descriptive of the respiratory movements of the subject as well as a respiratory signal 300 acquired using a respiratory sensor system comprising a below and a respiratory signal 300 acquired using a respiratory sensor system comprising an optical sensor are shown. A correlation between the respiratory signal 310 acquired using the below is 0.542. A correlation between the respiratory signal 310 acquired using the optical sensor is 0.988.

Fig. 6 shows further exemplary respiratory signals 310. These respiratory signals may be classified as shallow breathing with a very long exhale phase. In this example, the compliant respiratory phase starts, e.g., one second after the peak of the respiratory cycle and may have a length of 4 seconds. Depending on the imaging time required for an imaging data acquisition, the imaging data acquisition may, e.g., start automatically one second after peak detection. In case the imaging time exceeds the length of the compliant respiratory phase, the imaging parameters for the imaging data acquisition may be adjusted to the length of the compliant respiratory phase. The imaging time may, e.g., be reduced to 4 seconds. Alternatively, the imaging data acquisition may be split into 2 or 3 chunks of 4 seconds or less each. The imaging data acquisition may in particular be executed without requiring a breath-holding by the subject. In Fig. 6, a motion field descriptive of the respiratory movements of the subject as well as a respiratory signal 300 acquired using a respiratory sensor system comprising a below and a respiratory signal 300 acquired using a respiratory sensor system comprising an optical sensor are shown. A correlation between the respiratory signal 310 acquired using the below is 0.854. A correlation between the respiratory signal 310 acquired using the optical sensor is 0.965.

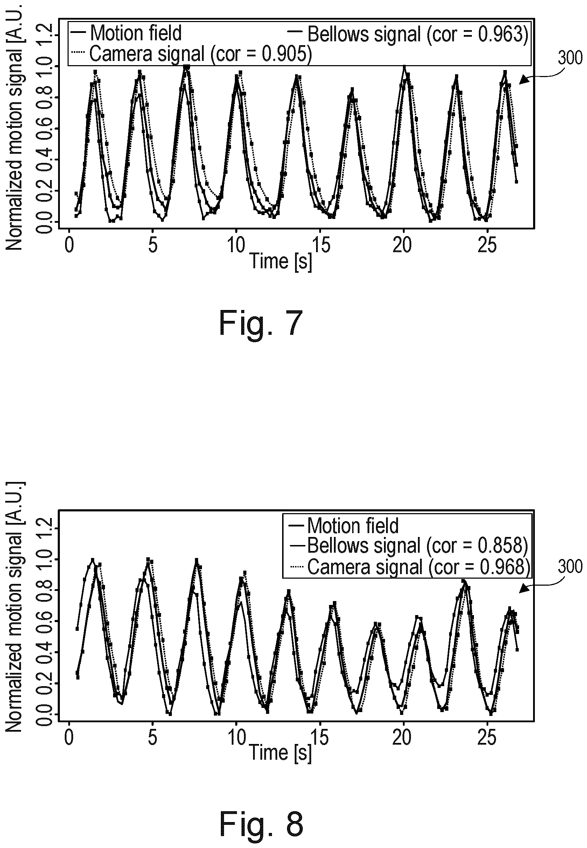

Fig. 7 and Fig. 8 show other exemplary respiratory signals 300, which may be classified as a medium-high-frequency breathing. The compliant respiratory phases of these respiratory may, e.g., be too short for an adjustment of the imaging data acquisition. For example, a length of the compliant respiratory phases may only be one second. An adjustment of the imaging parameters may, e.g., be executed as long as the imaging time exceeds the length of the compliant respiratory phase by a time span equal to or smaller than a predefined maximum time span. In response to determining that the imaging time exceeds the length of the compliant respiratory phase by a time span larger than a predefined maximum time span, the subject may, e.g., be instructed to hold his or her breath during the imaging data acquisition. For example, a breath-hold imaging data acquisition with auto-voice breath-hold commands may be executed. The auto-voice breath-hold commands may, e.g., be timed to the respiratory cycle of the respiratory signal. In Fig. 7 and Fig. 8, a motion field descriptive of the respiratory movements of the subject as well as a respiratory signal 300 acquired using a respiratory sensor system comprising a below and a respiratory signal 300 acquired using a respiratory sensor system comprising an optical sensor are shown. In case of Fig. 7, a correlation between the respiratory signal 310 acquired using the below is 0.963. A correlation between the respiratory signal 310 acquired using the optical sensor is 0.905. In case of Fig. 8, a correlation between the respiratory signal 310 acquired using the below is 0.858. A correlation between the respiratory signal 310 acquired using the optical sensor is 0.968.

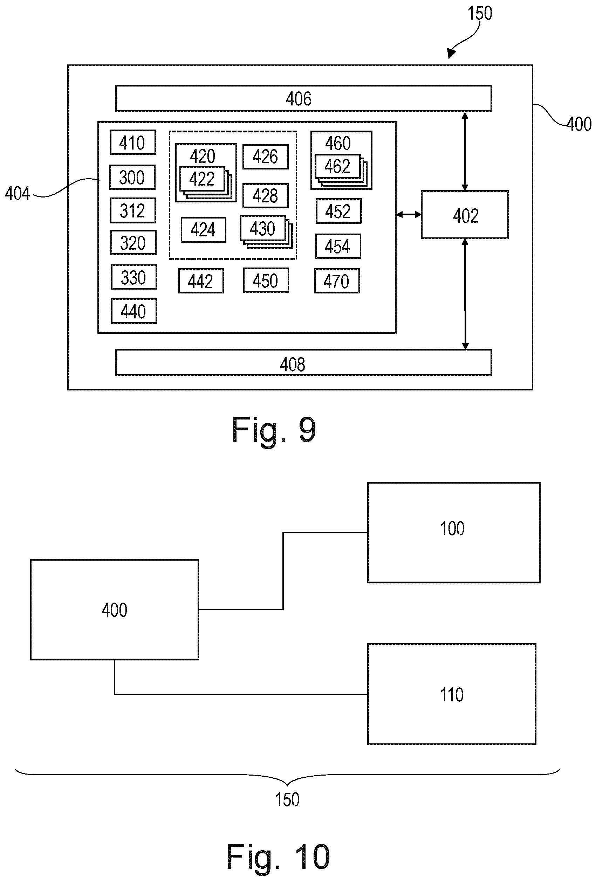

Fig. 9 shows an exemplary computational system 400 of an exemplary medical system 150. The computational system 400 may, e.g., be configured for controlling an imaging data acquisition by a medical imaging system from a region of interest of a subject, which is at least partially moved due to respiratory movements of the subject.

The computational system 400 is shown as comprising a computational component 402. The computational component 402 is intended to represent one or more processors or processing cores or other computational elements. The computational component 402 is shown as being connected to a hardware interface 406 and a memory 404. The hardware interface 406 may enable the computational component 402 to exchange commands and data with other components, e.g., a respiratory sensor system and/or a medical imaging system, like a CT system or an MRI system. The hardware interface 406 may, e.g., enable the computational component 402 to control the respiratory sensor system to acquire a respiratory signal 300. The hardware interface 406 may further, e.g., enable the computational component 402 to control a medical imaging system, like a CT system or an MRI system, to acquire imaging data 470.

The computational system 400 is further shown as being connected to a user interface 408 which may for example enable an operator to control and operate the computational system 400 and via the computational system 400, e.g., a respiratory sensor system and/or a medical imaging system. The user interface 408 may, e.g., comprise an output and/or input device enabling the operator to interact with the computational system 400. The output device may, e.g., comprise a display device configured for displaying an acquired respiratory signal 300. Furthermore, the display device may, e.g., be configured for displaying medical images generated using imaging data 470 acquired by the medical imaging system. The input device may, e.g., comprise a keyboard and/or a mouse enabling the operator to insert control commands for controlling the computational system 400 and via the computational system 400, e.g., the respiratory sensor system and/or the medical imaging system.

The memory 404 is shown as containing machine-executable instructions 410. The machine-executable instructions 410 enable the computational component 402 to perform controlling tasks, such as controlling a respiratory sensor system and/or a medical imaging system, to perform numerical tasks, as well as performing various signal data processing tasks, like analyzing the respiratory signal 300. The machine-executable instructions 410 may, e.g., enable the computational component 402 and thus the computational system 400 to execute any of the methods of Fig. 1 or 2.

The memory 404 is further shown as containing an imaging protocol 420 defining a set of imaging parameters 422 for an executing of an imaging data acquisition by a medical imaging system, like a CT system or an MRI system. The imaging protocol 420 is further shown to be assigned a predefined movement tolerance 424. The movement tolerance 424 defines a maximum level of movement acceptable for the imaging data acquisition. The imaging data acquisition is assigned with an imaging time 426 defining a time interval required for acquiring imaging data using the imaging parameters 422 according to the imaging protocol 420. The imaging protocol may further be assigned with a set of potential adjustments 430 of the imaging parameters 422 of the imaging protocol 420 for reducing the imaging time 426. The imaging protocol may further be assigned with compensable time span 428, which defines a maximum time span by which the imaging time 426 is reducible using the set of potential adjustments 430.

The memory 404 is further shown as containing a respiratory signal 300 of the subject, which may be acquired using a respiratory sensor system and which is descriptive of respiratory movements of a region of interest during respiratory cycles of the subject. The respiratory signal 300 may be analyzed and one or more of the following determined: a motion index 312 of respiratory movements of the region of interest, a compliant respiratory phase 320 within the individual respiratory cycles 310 of the respiratory signal 300 as well as a length 440 compliant respiratory phase 320; a trigger 330 for triggering an imaging data acquisition synchronously with a start of the compliant respiratory phase 320.

The memory 404 is further shown as containing adjustments 450, 453, 454 of the imaging parameters 422. These adjustments comprise, e.g., first adjustments 450 determined in response to determining that the imaging time 426 exceeds the length 440 of the compliant respiratory phase 310 by a time span equal to or smaller than a predefined time span 442. The first adjustments 450 are adjustments of the imaging parameters 422 for fitting the imaging data acquisition into a single compliant respiratory phase 320 of a single respiratory cycle. The first adjustments 450 of the imaging parameters 422 may, e.g., be selected from the potential adjustments 430.

The respiratory signal 300 may, e.g., be a spatially resolved signal descriptive of a spatial distribution of the respiratory movements within the region of interest over time. The imaging parameters 422 may define a spatial sequence of the imaging data acquisition within the region of interest and the determining of the first adjustments 450 of the imaging parameters 422 may comprise an adjusting of the spatial sequence of the imaging data acquisition depending on the spatial distribution of the respiratory movements.

The adjustments comprise, e.g., second adjustments 452 determined in response to determining that the imaging time 426 exceeds the length 440 of the compliant respiratory phase 310 by a time span larger than the predefined time span 442. The second adjustments 452 are adjustments of the imaging parameters 422 for splitting the imaging data acquisition into two or more imaging data acquisition sets to be acquired during two or more compliant respiratory phases 320 of two or more of the respiratory cycles.

The adjustments comprise, e.g., third adjustments 454 determined in response to determining that the imaging time 426 exceeds the length 440 of the compliant respiratory phase 310 by a time span larger than the predefined time span 442. The third adjustments 454 are adjustments of the imaging protocol 420 for fitting a maximum portion of the imaging data acquisition into a first acquisition set of the two or more acquisition sets acquired during a first compliant respiratory phase of a first respiratory cycle of the two or more respiratory cycles.

The memory 404 is further shown as containing an adjusted imaging protocol 460 with adjusted imaging parameters 462. The adjusted imaging parameters 462 may be imaging parameters 422 adjusted using one or more of the determined adjustments 450, 452, 454. The adjusted imaging protocol 460 with adjusted imaging parameters 462 may be used to control the medical imaging system to acquire imaging data 470 from the region of interest during one or more compliant respiratory phases 320 of one or more of the respiratory cycles. The imaging data 470 may, e.g., be used for reconstructing medical images.

Fig. 10 shows a schematic block diagram of an exemplary medical system 150. The medical system 150 comprises a computational system 400 for controlling an imaging data acquisition by a medical imaging system 110 from a region of interest of a subject with the region of interest being at least partially moved due to respiratory movements of the subject. The computational system 400 may, e.g., be configured to perform the method of any of the examples disclosed herein.

The medical system 150, e.g., further comprises a respiratory sensor system 100 for acquiring a respiratory signal of the subject. For example, the computational system 400 is further configured for controlling the respiratory sensor system 100. The medical system 150, e.g., further comprises a medical imaging system 110, e.g., a CT or MRI system. For example, the computational system 400 is further configured for controlling the medical imaging system 150. The computational system 400 may, e.g., be configured to use the respiratory signal acquired by the respiratory sensor system 100 for controlling an imaging data acquisition using the medical imaging system 110.

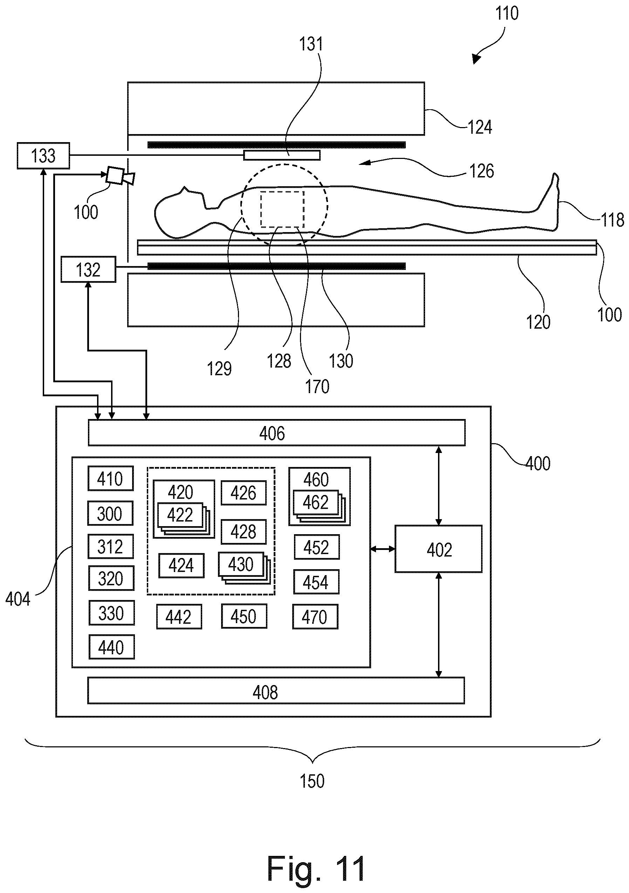

Fig. 11 shows a further exemplary medical system 150. The medical system 150 comprises a computational system 400 as shown in Fig. 9. Furthermore, the medical system 150 comprises a respiratory sensor system 100 for acquiring a respiratory signal of the subject 118. The respiratory sensor system 100 may, e.g., comprise an optical sensor and/or two-dimensional sensor array with a plurality of pressure sensors. The pressure sensors of the two-dimensional sensor array may be configured for detecting pressure changes over time due to motions of a subject 118 lying on the sensor array. The sensor array may, e.g., be embedded into a mattress. Alternatively or additionally, also other types of respiratory sensors may be comprised by the respiratory sensor system 100.

Furthermore, the medical system 150 comprises a medical imaging system 110 in form of an MRI or magnetic resonance imaging system. The MRI system 110 is shown as being controlled by the computational system 400. The hardware interface 406 allows a computational component 402 of the computational system 400, e.g., a processor, to exchange data with and/or control the MRI system 110.

The MRI system 110 may, e.g., comprise a magnet 124. Magnet 124 is a superconducting cylindrical type of magnet with a bore 126 through it. The use of different types of magnets is also possible. For instance, it is also possible to use both a split cylindrical magnet and a so-called open magnet. A split cylindrical magnet is similar to a standard cylindrical magnet, except that the cryostat has been split into two sections to allow access to the iso-plane of the magnet. Such magnets may, for instance, be used in conjunction with charged particle beam therapy. An open magnet has two magnet sections, one above the other with a space in-between that is large enough to receive a subject: the arrangement of the two sections is similar to that of a Helmholtz coil. Open magnets are popular because the subject is less confined. Inside the cryostat of the cylindrical magnet there is a collection of superconducting coils.

Within bore 126 of the cylindrical magnet 124 there is an imaging zone 129, where the magnetic field is strong and uniform enough to perform magnetic resonance imaging. A field of view 128 is shown within imaging zone 129. The MRI data 470 is typically acquired for the field of view 128 comprising the region 170 of interest of a subject 118. The subject 118 is shown as being supported by a subject support 120 such that at least a portion of subject 118 is within the imaging zone 129 and the field of view 128.

Within the bore 126 of the magnet there is also a set of magnetic field gradient coils 130 which is used to spatially encode magnetic spins within the bore 126 of the magnet 124. The magnetic field gradient coils 130 are connected to a gradient controller, i.e., a magnetic field gradient coil power supply 132. The magnetic field gradient coils 130 are intended to be representative. Typically, magnetic field gradient coils 130 contain three separate sets of coils for spatial encoding in three orthogonal spatial directions. The magnetic field gradient power supply 132 supplies current to the magnetic field gradient coils 130. The current supplied to the magnetic field gradient coils 130 is controlled as a function of time and may be ramped or pulsed.

Adjacent to the imaging zone 129 is a radio-frequency antenna 131 arranged for manipulating the orientations of magnetic spins within the imaging zone 129 and for receiving radio transmissions from spins also within the imaging zone 129. The radio-frequency antenna 131 may contain multiple coil elements. The radio-frequency antenna 131 is connected to a radio-frequency transceiver 133. The radio-frequency antenna 131 and radio-frequency transceiver 133 may be replaced by separate transmit and receive coils and a separate transmitter and receiver. It is understood that the radio-frequency antenna 131 and the radio-frequency transceiver 133 are representative. The radio-frequency antenna 131 is intended to also represent a dedicated transmit antenna and a dedicated receive antenna. Likewise, the transceiver 133 may also represent a separate transmitter and receivers. The radio-frequency antenna 131 may also have multiple receive/transmit elements and the radio-frequency transceiver 133 may have multiple receive/transmit channels. The transceiver 133 and the gradient controller 132 are shown as being connected to the hardware interface 406 of the computational system 400.

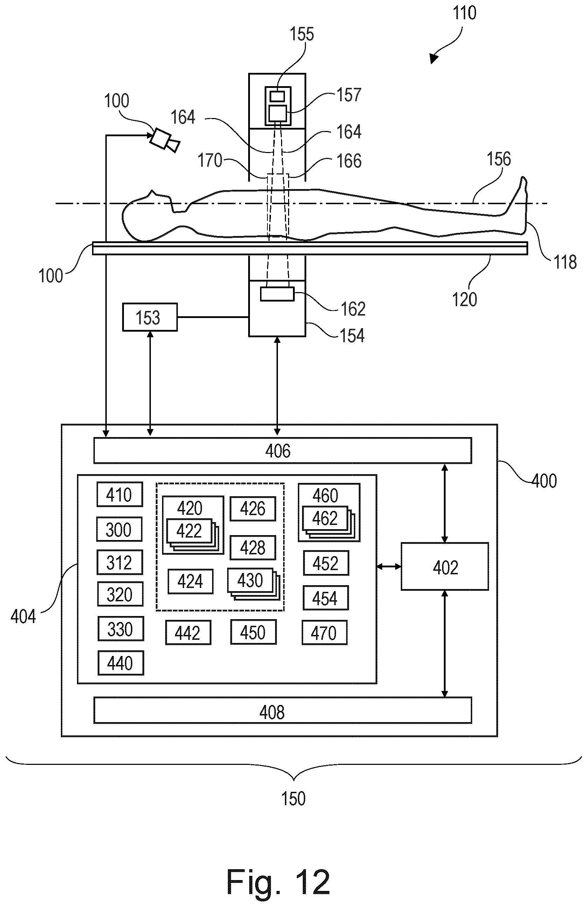

Fig. 12 shows a further exemplary medical system 150. The medical system 150 comprises a computational system 400 as shown in Fig. 9. Furthermore, the medical system 150 comprises a respiratory sensor system 100 for acquiring a respiratory signal of the subject 118. The respiratory sensor system 100 may, e.g., comprise an optical sensor and/or two-dimensional sensor array with a plurality of pressure sensors. The pressure sensors of the two-dimensional sensor array may be configured for detecting pressure changes over time due to motions of a subject 118 lying on the sensor array. The sensor array may, e.g., be embedded into a mattress. Alternatively or additionally, also other types of respiratory sensors may be comprised by the respiratory sensor system 100.

Furthermore, the medical system 150 comprises a medical imaging system 110 in form of a CT or computed tomography system. The CT system 110 is shown as being controlled by the computational system 400. The hardware interface 406 allows a computational component 402 of the computational system 400, e.g., a processor, to exchange data with and/or control the CT system 110.

The CT system 110 may, e.g., comprise a rotating gantry 154. The gantry 154 rotates about an axis of rotation 156. There is a subject 118 lying on a subject support 120. On the subject support 120 is the two-dimensional sensor array 100 arranged for detecting pressure changes over time due to motions of a subject 118 lying on the sensor array 100. Within gantry 154 is an X-ray tube 157 placed, e.g., within an X-ray tube high voltage isolation tank. In addition, a voltage stabilizer circuit 155 may, e.g., be provided with the X-ray tube 157, within an X-ray power supply 153 or external to both. The X-ray power supply 153 supplies the X-ray tube 157 with power.

X-ray tube 157 produces X-rays 164 that pass through the subject 118 and are received by a detector 162. Within the area of box 166 is an imaging zone comprising the region 170 of interest of the subject 118, from where CT imaging data 470 of the subject 118 can be acquired. This imaging data 470 may be used to reconstruct CT images.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer-readable medium(s) having computer executable code embodied thereon.

Any combination of one or more computer-readable medium(s) may be utilized. The computer-readable medium may be a computer-readable signal medium or a computer-readable storage medium. A 'computer-readable storage medium" as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer-readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer-readable-storage medium also refers to various types of recording media capable of being accessed by the computer device via a network or communication link. For example, data may be retrieved over a modem, over the internet, or over a local area network. Computer executable code embodied on a computer-readable medium may be transmitted using any appropriate medium, including but not limited to wireless, wire line, optical fiber cable, RF, etc., or any suitable combination of the foregoing.

A computer-readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer-readable signal medium may be any computer-readable medium that is not a computer-readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage" or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine-executable instruction or computer executable code. References to the computational system comprising the example of a 'computational system' should be interpreted as possibly containing more than one computational component or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine-executable code or instructions may be executed by multiple computational systems or processors that may be within the same computing device or which may even be distributed across multiple computing devices.

Machine-executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++, or the like and conventional procedural programming languages, such as the "C" programming language or similar programming languages and compiled into machine-executable instructions. In some instances, the computer executable code may be in the form of a high-level language or in a pre-compiled form and be used in conjunction with an interpreter which generates the machine-executable instructions on the fly. In other instances, the machine-executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine-executable instructions or computer program instructions may also be stored in a computer-readable medium that can direct a computer, other programmable data processing apparatus, or other devices to function in a particular manner, such that the instructions stored in the computer-readable medium produce an article of manufacture including instructions which implement the function/act specified in the flowchart and/or block diagram block or blocks.

The machine-executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device." A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode Ray Tube (CRT), storage tube, bi-stable display, electronic paper, vector display, flat panel display, Vacuum Fluorescent (VF) display, Light-Emitting Diode (LED) displays, Electroluminescent display (ELD), Plasma Display Panels (PDP), Liquid Crystal Display (LCD), Organic Light-Emitting Diode (OLED) displays, a projector, and head-mounted display.

Magnetic resonance imaging data is defined herein as being recorded measurements of RF signals emitted by atomic spins using the antenna of a magnetic resonance apparatus, like a magnetic resonance imaging system. A magnetic resonance image is defined herein as being the reconstructed twoor three-dimensional visualization of anatomic data comprised by the magnetic resonance imaging data.

Computed tomography imaging data is defined herein as being the recorded X-ray measurements taken from a variety of angles. A rotating X-ray tube and a row of detectors placed in a gantry to measure X-ray attenuations by different tissues inside a subject may be used for executing the X-ray measurements from a variety of angles. A computed tomography image is defined herein as being the reconstructed two- or three-dimensional visualization of anatomic data comprised by the computed tomography imaging data.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to an advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: respiratory sensor system
- 110: medical imaging system
- 118: subject
- 120: support
- 124: magnet
- 126: bore of magnet
- 128: field of view
- 129: imaging zone
- 130: magnetic field gradient coils
- 131: radio-frequency antenna
- 132: gradient controller
- 133: radio-frequency transceiver
- 150: medical system
- 153: X-ray power supply
- 154: gantry
- 155: voltage stabilizer circuit
- 156: axis of rotation
- 157: X-ray tube
- 162: detector
- 164: X-rays
- 166: imaging zone
- 170: region of interest
- 200: receiving an imaging protocol defining a set of imaging parameters
- 202: receiving a respiratory signal
- 204: analyzing the respiratory signal
- 206: comparing a length of a compliant respiratory phase with an imaging time
- 208: imaging time exceeding the length of the compliant respiratory phase?
- 210: adjusting the imaging protocol
- 212: controlling the medical imaging system using the adjusted imaging protocol
- 214: controlling the medical imaging system using the imaging protocol
- 216: controlling the medical imaging system using the imaging protocol without adjustments
- 220: determining a start time of compliant respiratory phases within respiratory cycles
- 222: determining a trigger for triggering the imaging data acquisition
- 224: monitoring the respiratory signal
- 226: trigger detected?
- 228: triggering an imaging data acquisition
- 300: respiratory signal
- 302: peak
- 304: peak
- 310: respiratory cycle
- 312: motion index
- 320: compliant respiratory phase
- 330: trigger
- 400: computational system
- 402: computational component
- 404: memory
- 406: hardware interface
- 408: user interface
- 410: machine readable instructions
- 420: imaging protocol
- 422: imaging parameters
- 424: movement tolerance
- 426: imaging time
- 428: compensable time span
- 430: potential adjustments
- 440: length of compliant respiratory phase
- 442: predefined time span
- 450: first adjustments
- 452: second adjustments
- 454: third adjustments
- 460: adjusted imaging protocol
- 462: adjusted imaging parameters
- 470: imaging data

## Claims

1. A method for controlling an imaging data acquisition by a medical imaging system (110) from a region of interest (170) of a subject (118) using a computational system (400), the region of interest (170) being at least partially moved due to respiratory movements of the subject (118), the method comprising:
- receiving an imaging protocol (420) defining a set of imaging parameters (422) for an executing of the imaging data acquisition by the medical imaging system, the imaging protocol (420) further being assigned with a predefined movement tolerance (424), the movement tolerance (424) defining a maximum level of movement acceptable for the imaging data acquisition, the imaging data acquisition being assigned with an imaging time (426) defining a time interval required for acquiring imaging data (470);
- receiving a respiratory signal (300) of the subject (118) descriptive of the respiratory movements of the region of interest (170) during respiratory cycles (310) of the subject (118);
- analyzing the respiratory signal (300), the analyzing comprising determining a motion index (312) of respiratory movements of the region of interest (170) and, using the motion index, a length (440) of a compliant respiratory phase (320) within the individual respiratory cycles (310), during which the respiratory movements of the region of interest (170) comply with the predefined movement tolerance (424);
- comparing the length (440) of the compliant respiratory phase (320) with the imaging time (426);
- controlling the medical imaging system (110) to acquire the imaging data (470) from the region of interest (170) based on a result of the comparing.

2. The method of claim 1, in response to determining that the imaging time (426) is equal to or smaller than the length (440) of the compliant respiratory phase (320), the controlling of the medical imaging system (110) to acquire the imaging data (470) from the region of interest (170) being a controlling to acquire the imaging data (470) during a single compliant respiratory phase (320) using the imaging protocol (420) with the imaging parameters (422).

3. The method of claim 1, in response to determining that the imaging time (426) exceeds the length (440) of the compliant respiratory phase (320), the method further comprising:
- determining adjustments (450, 452, 454) of the imaging parameters (422) for adjusting the imaging data acquisition to the length (440) of the compliant respiratory phase (320);
- adjusting the imaging protocol (420), the adjusting comprising adjusting imaging parameters (462) of the imaging protocol (420) using the determined adjustments (450, 452, 454);
the controlling of the medical imaging system (110) to acquire the imaging data (470) from the region of interest (170) being a controlling to acquire the imaging data (470) during one or more compliant respiratory phases (320) of one or more of the respiratory cycles (310) using the adjusted imaging protocol (460) with the adjusted imaging parameters (462).

4. The method of claim 3, in response to determining that the imaging time (426) exceeds the length (440) of the compliant respiratory phase (320) by a time span (442) equal to or smaller than a predefined time span (442), the adjustments being determined comprising first adjustments (450) of the imaging parameters (422) for fitting the imaging data acquisition into a single compliant respiratory phase (320) of a single respiratory cycle (310), the controlling of the medical imaging system (110) to acquire the imaging data (470) from the region of interest (170) being a controlling to acquire the imaging data (470) during a single compliant respiratory phase (320).

5. The method of claim 4, the imaging protocol (420) further being assigned with a set of potential adjustments (430) of the imaging parameters (422) of the imaging protocol (420) for reducing the imaging time (426), the determining of the first adjustments (450) of the imaging parameters (422) comprising selecting one or more of the potential adjustments (430) from the set of potential adjustments (430).

6. The method of claim 5, the selecting comprising using a selection criterion, the selecting criterion requiring a reduction of the imaging time (426) resulting from using the selected potential adjustments (430) to be a minimum reduction sufficient to fit the reduced imaging time (426) into the single compliant respiratory phase (320).

7. The method of any of claims 5 to 6, the medical imaging system (110) comprising a computed tomography system, the imaging data acquisition being a computed tomography data acquisition, the imaging parameters (422) being adjusted for reducing the imaging time (426) comprising one or more of the following: a gantry rotation speed; a table pitch; a collimation size; a scan length; or
the medical imaging system (110) comprising a magnetic resonance imaging system, the imaging data acquisition being a magnetic resonance imaging data acquisition, the imaging parameters (422) being adjusted for reducing the imaging time (426) comprising one or more of the following: an acquisition matrix size; a sampling rate; a field of view.

8. The method of any of claims 4 to 7, the respiratory signal (300) being a spatially resolved signal (300) descriptive of a spatial distribution of the respiratory movements within the region of interest (170) over time, the imaging parameters (422) defining a spatial sequence of the imaging data acquisition within the region of interest (170), the determining of the first adjustments (450) of the imaging parameters (422) comprising an adjusting of the spatial sequence of the imaging data acquisition defined by the imaging parameters (422) depending on the spatial distribution of the respiratory movements.

9. The method of any of claims 3 to 8, in response to determining that the imaging time (426) exceeds the length (440) of the compliant respiratory phase (320) by a time span (442) larger than a predefined time span (442), the adjustments being determined comprising second adjustments (452) of the imaging parameters (422) for splitting the imaging data acquisition into two or more imaging data acquisition sets to be acquired during two or more compliant respiratory phases (320) of two or more of the respiratory cycles (310), each of the acquisition sets being configured to require an execution time fitting into one of the respective compliant respiratory phases (320), the controlling of the medical imaging system (110) to acquire the imaging data (470) from the region of interest (170) being a controlling to acquire the imaging data (470) during the two or more compliant respiratory phases (320) of the two or more respiratory cycles (310).

10. The method of claim 9, in response to the determining that the imaging time (426) exceeds the length (440) of the compliant respiratory phase (320) by a time span (442) larger than the predefined time span (442), the adjustments being determined additionally comprising third adjustments (454) of the imaging parameters (422) of the imaging protocol (420) for fitting a maximum portion of the imaging data acquisition into a first acquisition set of the two or more acquisition sets acquired during a first compliant respiratory phase (320) of a first respiratory cycle (310) of the two or more respiratory cycles (310).

11. The method of any claims 4 to 10, the predefined time span (442) being one of the following: a predefined fraction of the imaging time (426); a compensable time span (428) assigned to the imaging protocol (420), which defines a maximum time span by which the imaging time (426) being reducible using the set of potential adjustments (430) of the imaging parameters (422) of the imaging protocol (420).

12. The method of any of claims 3 to 11, the analyzing of the respiratory signal (300) further comprising:
- determining a start time of the compliant respiratory phases (320) within the respiratory cycles (310);
- determining, using the start time, a trigger (330) comprised by the respiratory cycles (310) within the respiratory signal (300) for triggering the imaging data acquisition in time with the start of the compliant respiratory phase (320);
- the controlling of the medical imaging system (110) comprising monitoring the respiratory signal (300) and triggering the acquiring of the imaging data (470) in response to detecting the trigger (330) in the respiratory signal (300).

13. A computer program for controlling an imaging data acquisition by a medical imaging system (110) from a region of interest (170) of a subject (118) with the region of interest (170) being at least partially moved due to respiratory movements of the subject (118), the computer program comprising machine executable instructions (410), an execution of the machine executable instructions (410) using a computational system (400) causing the computational system (400) to perform the method of any of the previous claims.

14. A medical system (150) comprising a computational system (400) for controlling an imaging data acquisition by a medical imaging system (110) from a region of interest (170) of a subject (118) with the region of interest (170) being at least partially moved due to respiratory movements of the subject (118), the computational system (400) comprising a memory (404) storing machine executable instructions (410) for an execution by a computational system (400), the execution of the machine executable instructions (410) causing the computational system (400) to perform the method of any of claims 1 to 12.

15. The medical system (150) of claim 14, the medical system (150) further comprising one or more of the following:
- the medical imaging system (110);
- a respiratory sensor system (100) configured for acquiring the respiratory signal (300) of the subject (118).
